# EUROPEAN PATENT APPLICATION

(11) **EP 4 559 925 A1**
(43) Date of publication of application: **28.05.2025**
(21) Application number: 23843060.7
(22) Date of filing: 21.07.2023
(51) Int. Cl.: C07H 21/00, C07H 19/10

(54) **PRODUCTION METHOD OF OLIGONUCLEOTIDE**

(30) Priority: 22.07.2022 JP 2022117422
(71) Applicant: Sumitomo Chemical Company, Limited, Tokyo 103-6020 (JP)
(72) Inventor: TAKESHITA, Reo, Oita-shi, Oita 870-0106 (JP); KANO, Toshifumi, Osaka-shi, Osaka 555-0021 (JP); TANAKA, Yuki, Oita-shi, Oita 870-0106 (JP)
(74) Representative: Mewburn Ellis LLP
(86) International application number: PCT/JP2023/026733
(87) International publication number: WO 2024/019137

(57) **Abstract**

The present invention relates to a production method of an oligonucleotide that includes a step of coupling a compound represented by formula (2) [wherein G² represents a protecting group of a hydroxy group, B^{a} represents a nucleobase which may be protected by a protecting group, R represents a protected hydroxy group or the like, X represents an oxygen atom or the like, and the bond marked with * represents a bond to the 3' terminal side of a nucleic acid.] with 2'-OMe-U amidite represented by formula (4) [wherein G¹ represents a protecting group of a hydroxy group.] in the presence of an activator, wherein the 2'-OMe-U amidite is dissolved in a mixed solution containing acetonitrile and an aromatic hydrocarbon represented by formula (3) [wherein Y¹ to Y⁶ represent a hydrogen atom, a methyl group, or the like.].

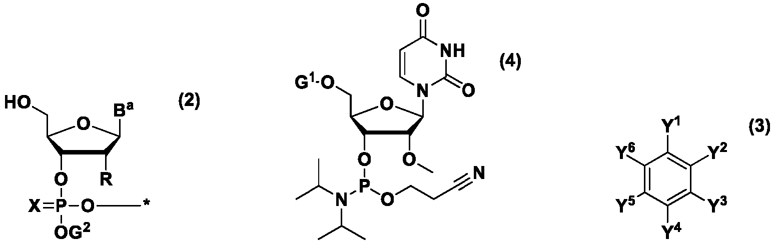

## Description

### TECHNICAL FIELD

This application claims priority to and the benefit of Japanese Patent Application No. 2022-117422 filed July 22, 2022 according to the Paris Convention, the entire contents of which are incorporated herein by reference.

The present invention relates to a production method of an oligonucleotide using the phosphoramidite method.

### BACKGROUND ART

Oligonucleotides, which are nucleic acid oligomers, are useful materials utilized in various fields such as nucleic acid probes for gene detection, DNA probes used in PCR, antisense nucleic acids, siRNA, and aptamers as pharmaceuticals.

Oligonucleotides can be produced by solid-phase synthesis method using the phosphoramidite method. In the phosphoramidite method, a phosphoramidite (hereinafter referred to as an "amidite") of a nucleoside, which is a monomer, is sequentially linked on a glass or a resin support to produce an oligonucleotide with desired sequences. The specific production step includes a deprotection step of deprotecting a protecting group of a hydroxy group at a 5' terminal of a nucleotide using an acidic solution, a coupling step of reacting the deprotected hydroxy group at the 5' terminal of the nucleotide with an amidite group of an amidite in the presence of an activator, an oxidation step of oxidizing the newly formed bond between nucleosides from a trivalent phosphorus to a pentavalent phosphorus, and a capping step of acylating an unreacted hydroxy group at the 5' terminal. By repeating these steps sequentially, amidites are linked in any desired order, and an oligonucleotide with the desired sequence is produced.

As disclosed in Patent Document 1 and the like, amidites used in the coupling step are dissolved in acetonitrile and used as amidite solutions. On the other hand, in the case of amidites with low solubility in acetonitrile, it is difficult to use this method.

2'-OMe-U amidite is exemplified as one of amidites with low solubility in acetonitrile. To improve the solubility of 2'-OMe-U amidite, it has been reported that an amidite solution is prepared by adding dichloromethane (Patent Literature 2, Non-Patent Literature 1) in addition to acetonitrile. By adding these solvents, the solubility of 2'-OMe-U amidite is improved.

Although such production methods of oligonucleotides have been developed, the purity of the oligonucleotides synthesized has not always been satisfactory. One of the reasons for this is the presence of n-1 mer impurities. N-1 mer impurities are impurities that formed during the production of oligonucleotides and have a chain length that is one unit shorter compared to the target chain length. It is known that these n-1 mer impurities are difficult to be separated from oligonucleotides with the target chain length (Non-Patent Literature 2). Therefore, reducing n-1 mer impurities that formed during production is important for the efficient production of oligonucleotides.

### CITATION LIST

### PATENT LITERATURE

Patent Literature 1: WO 2019/170731 A1
Patent Literature 2: CN 112007040 A description

### NON-PATENT LITERATURE

Non-Patent Literature 1: Bioorganic & Medicinal Chemistry Letters 28 (2018) 3774-3779
Non-Patent Literature 2: Mass Spectrometry Reviews 40 (2021) 75-109

### SUMMARY OF THE INVENTION

### PROBLEMS TO BE SOLVED BY INVENTION

An object of the present invention is to provide a high-purity production method of an oligonucleotide containing one or more 2'-OMe-U.

### MEANS TO SOLVE PROBLEMS

The present inventors have intensively studied to achieve the above object and have provided a nucleic acid production method characterized by using acetonitrile and an aromatic hydrocarbon as solvents for a 2'-OMe-U amidite solution.

The present invention encompasses the following aspects but are not limited thereto.
1. A production method of an oligonucleotide comprising a step of coupling a compound represented by formula (2): 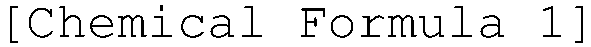 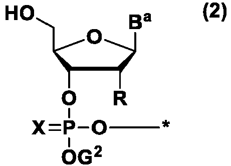 (wherein,
   G² represents a protecting group of a hydroxy group,
   B^{a} represents a nucleobase which may be protected by a protecting group,
   R represents a protected hydroxy group, a hydrogen atom, a fluorine atom, a methoxy group, a 2-methoxyethyl group, or an OQ' group,
   Q' represents an alkylene group which is bonded to the carbon atom at the 4' position of the ribose,
   X represents an oxygen atom or a sulfur atom, and
   the bond marked with * represents a bond to the 3' terminal side of a nucleic acid.)
   with 2'-OMe-U amidite represented by formula (4): 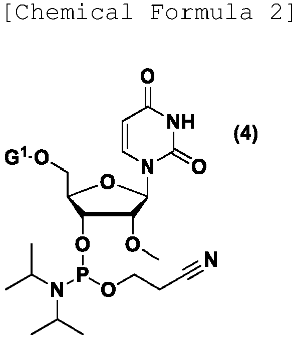 (wherein,
   G¹ represents a protecting group of a hydroxy group.) in the presence of an activator, wherein the 2'-OMe-U amidite is dissolved in a mixed solution containing acetonitrile and an aromatic hydrocarbon represented by formula (3): 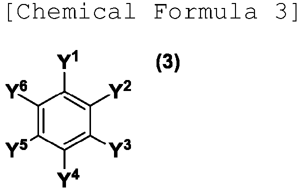 (wherein, Y¹ to Y⁶ are identical to or different from each other and each independently represents a hydrogen atom, a methyl group, an ethyl group, or a halogen atom.).
2. The production method of the oligonucleotide according to [1] wherein the aromatic hydrocarbon represented by formula (3) is benzene having one or two substituents selected from the group consisting of a methyl group, an ethyl group, and a halogen atom, and when benzene has two substituents, the substituents may be the same or different.
3. The production method of the oligonucleotide according to [1] wherein the aromatic hydrocarbon represented by formula (3) is selected from the group consisting of toluene, *o*-xylene, *m*-xylene, *p*-xylene, chlorobenzene, *o-*dichlorobenzene, *m*-dichlorobenzene, *p*-dichlorobenzene, and mixtures of two or more of these.
4. The production method of the oligonucleotide according to any one of [1] to [3] wherein the volume ratio of acetonitrile to the aromatic hydrocarbon represented by formula (3) in the mixed solution in which 2'-OMe-U amidite represented by formula (4) is dissolved is 99:1 to 1:99 in acetonitrile: the aromatic hydrocarbon represented by formula (3).
5. The production method of the oligonucleotide according to any one of [1] to [3] wherein the volume ratio of acetonitrile to the aromatic hydrocarbon represented by formula (3) in the mixed solution in which 2'-OMe-U amidite represented by formula (4) is dissolved is 90:10 to 10:90 in acetonitrile: the aromatic hydrocarbon represented by formula (3).
6. The production method of the oligonucleotide according to any one of [1] to [3] wherein the volume ratio of acetonitrile to the aromatic hydrocarbon represented by formula (3) in the mixed solution in which 2'-OMe-U amidite represented by formula (4) is dissolved is 90:10 to 40:60 in acetonitrile: the aromatic hydrocarbon represented by formula (3).
7. The production method of the oligonucleotide according to any one of [1] to [6] wherein the concentration of 2'-OMe-U amidite in the 2'-OMe-U amidite solution represented by formula (4) is 0.01 to 0.4 M.
8. The production method of the oligonucleotide according to any one of [1] to [6] wherein the concentration of 2'-OMe-U amidite in the 2'-OMe-U amidite solution represented by formula (4) is 0.05 to 0.2 M.
9. The production method of the oligonucleotide according to any one of [1] to [8] wherein the activator is 5-benzylthio-1H-tetrazole.
10. The production method of the oligonucleotide according to any one of [1] to [9] wherein when R in the compound represented by formula (2) is a protected hydroxy group, the protecting group of the hydroxy group is a protecting group represented by formula (10): 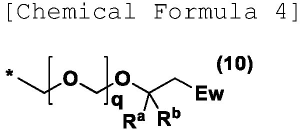 (wherein,
   q represents an integer from 0 to 5,
   R^{a} and R^{d} are identical to or different from each other and each represents a methyl group, an ethyl group, or a hydrogen atom,
   the bond marked with * is bonded to the oxygen of the 2' hydroxy group, and
   Ew represents an electron-withdrawing group.).
11. The production method of the oligonucleotide according to any one of [1] to [10] wherein in 2'-OMe-U amidite represented by formula (4), G¹ is a 4,4'-dimethoxytrityl group.
12. The production method of the oligonucleotide according to any one of [1] to [11] conducted by solid-phase synthesis method.
13. A solution containing 2'-OMe-U amidite represented by formula (4): 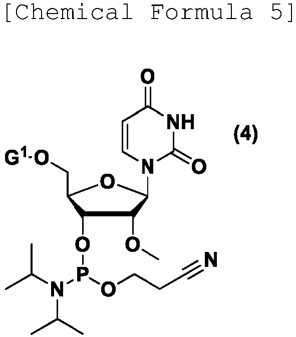 (wherein,
   G¹ represents a protecting group of a hydroxy group.) acetonitrile, and an aromatic hydrocarbon represented by formula (3): 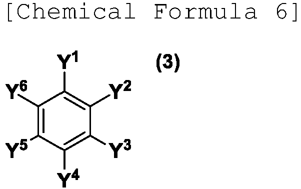 (wherein, Y¹ to Y⁶ are identical to or different from each other and each independently represents a hydrogen atom, a methyl group, an ethyl group, or a halogen atom.). 14. A method of using the solution described in [13] containing 2'-OMe-U amidite represented by formula (4), acetonitrile, and the aromatic hydrocarbon represented by formula (3) for a production of an oligonucleotide.

### Effect of Invention

The present invention provides a high-purity production method of an oligonucleotide containing one or more 2'-OMe-U amidite.

### BRIEF DESCRIPTION OF THE DRAWINGS

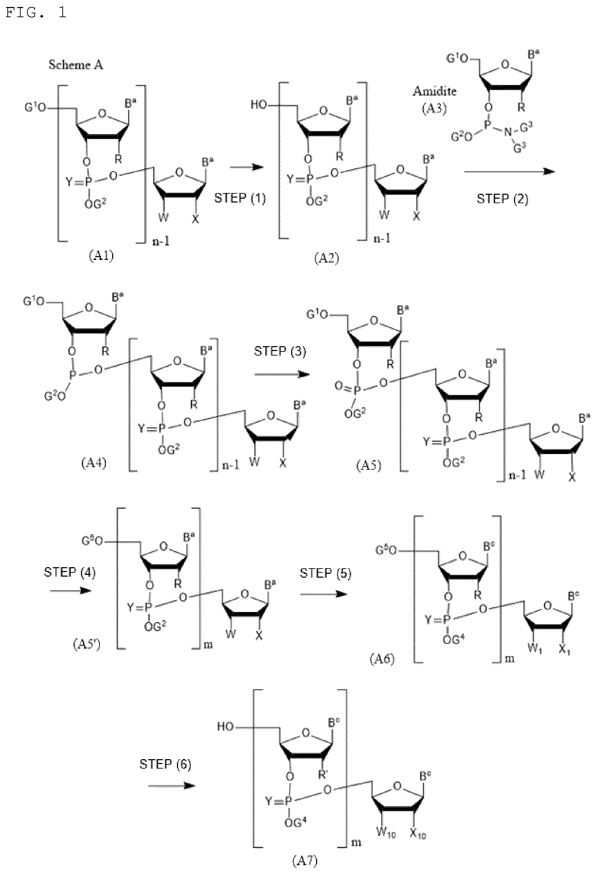

[Figure 1] is a drawing showing the scheme (Scheme A) of steps (1) to (6) of the production method of the present invention.

### MODE FOR CARRYING OUT THE INVENTION

The production method of an oligonucleotide of the present invention involves:
A production method of an oligonucleotide comprising a step of coupling a compound represented by formula (2): 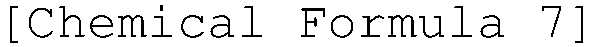 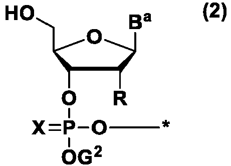 (wherein,
G² represents a protecting group of a hydroxy group,
B^{a} represents a nucleobase which may be protected by a protecting group,
R represents a protected hydroxy group, a hydrogen atom, a fluorine atom, a methoxy group, a 2-methoxyethyl group, or an OQ' group,
Q' represents an alkylene group which is bonded to the carbon atom at the 4' position of the ribose,
X represents an oxygen atom or a sulfur atom, and
the bond marked with * represents a bond to the 3' terminal side of a nucleic acid.)
with 2'-OMe-U amidite represented by formula (4): 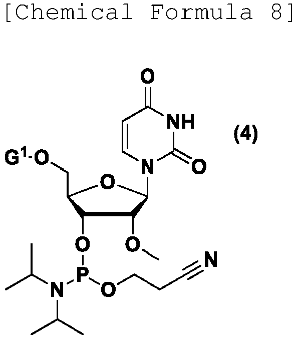 (wherein,
G¹ represents a protecting group of a hydroxy group.) in the presence of an activator, wherein the 2'-OMe-U amidite is dissolved in a mixed solution containing acetonitrile and an aromatic hydrocarbon represented by formula (3): 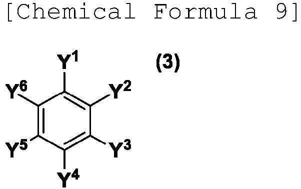 (wherein, Y¹ to Y⁶ are identical to or different from each other and each independently represents a hydrogen atom, a methyl group, an ethyl group, or a halogen atom.).

The production method of the oligonucleotide may include a step of reacting a 2'-OMe-U amidite solution, in which 2'-OMe-U amidite represented by formula (4) is dissolved in a mixed solution of acetonitrile and an aromatic hydrocarbon represented by formula (3), with an activator. The following explains the production method of the oligonucleotide.

In the solvent of the 2'-OMe-U amidite solution in the present invention, by using a mixed solution containing acetonitrile and an aromatic hydrocarbon represented by formula (3), it is possible to reduce the n-1 mer contained in the oligonucleotide produced. The mixing ratio in the mixed solution containing acetonitrile and the aromatic hydrocarbon represented by formula (3) is not particularly limited, but the volume ratio of acetonitrile to the aromatic hydrocarbon is, for example, 99:1 to 1:99, preferably 90:10 to 10:90, more preferably 90:10 to 40:60.

As the aromatic hydrocarbon, benzene having substituents selected from the group consisting of a methyl group, an ethyl group, or a halogen atom, preferably benzene having one or two substituents selected from the group consisting of a methyl group, an ethyl group, or a halogen atom, more preferably toluene, o-xylene, m-xylene, p-xylene, chlorobenzene, o-dichlorobenzene, m-dichlorobenzene, p-dichlorobenzene, and mixtures of two or more of these can be used.

Solvents other than acetonitrile and an aromatic hydrocarbon may also be mixed and used in the 2'-OMe-U amidite solution.

The concentration of 2'-OMe-U amidite in the 2'-OMe-U amidite solution is preferably adjusted to 0.01 M to 0.4 M, more preferably to 0.05 M to 0.2 M.

It is also possible to use 2'-OMe-U amidite after dissolving it in the aforementioned mixed solvent and then reducing the water content with a drying agent such as zeolum.

For storing the 2'-OMe-U amidite solution, a glass container, a plastic container, or a metal container can be used. As the plastic container, a container made of polyethylene or polypropylene can be used, and as the metal container, a container made of SUS or Hastelloy can be used.

A reaction precursor with 2'-OMe-U amidite represented by formula (4) is specifically a compound represented by the following formula (12): 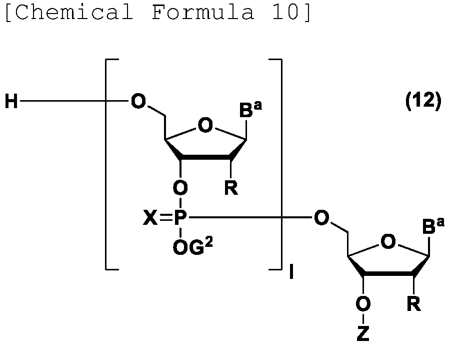 (wherein,
G² represents a protecting group of a hydroxy group,
B^{a} each independently represents a nucleobase which may be protected by a protecting group,
R represents a protected hydroxy group, a hydrogen atom, a fluorine atom, a methoxy group, a 2-methoxyethyl group, or an OQ' group,
Q' represents an alkylene group which is bonded to the carbon atom at the 4' position of the ribose,
X is identical to or different from each other and each independently represents an oxygen atom or a sulfur atom,
Z represents a group consisting of a solid support and a connection group connecting the solid support with the oxygen atom of the 3' hydroxy group at the 3' terminal ribose of the oligonucleotide, and
1 represents an integer from 0 to 300.)
by reacting with 2'-OMe-U amidite, it is converted into an oligonucleotide represented by formula (13): 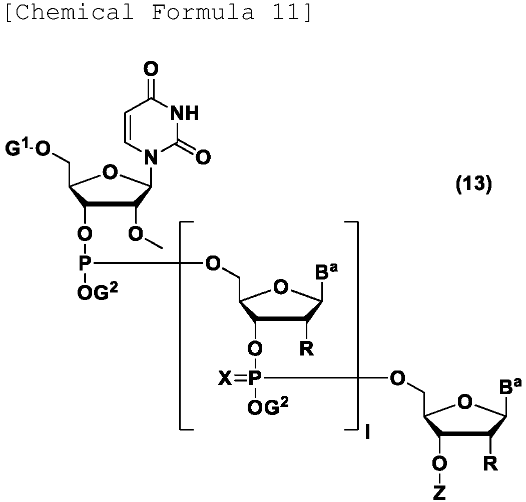 (wherein,
G¹ represents a protecting group of a hydroxy group,
G², B^{a}, R, X, Z, and 1 are as defined above.)

In formulas (2), (12), and (13), when R represents an OQ' group and Q' represents an alkylene group which is bonded to the carbon atom at the 4' position of ribose, specific examples of such structures include the following LNA-1 to LNA-4. 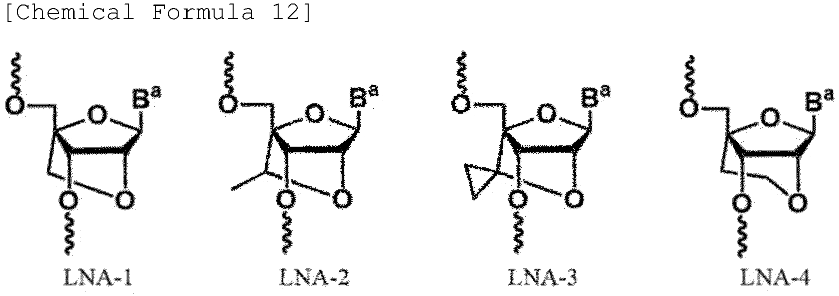 (wherein, B^{a} represents a nucleobase which may be protected.)

The nucleosides (ribose and deoxyribose) contained in the oligonucleotides used in the present invention include DNA, RNA, 2'-O-MOE (2'-O-methoxyethyl), 2'-O-Me, 2'-F RNA, and the aforementioned LNA, but are not limited to these.

As the group represented by Z, which consists of a solid support and a connection group connecting the solid support with the oxygen atom of the 2' or 3' hydroxy group at the 3' terminal ribose of the nucleic acid oligomer, more specifically, a structure represented by the following formula (18) can be listed.

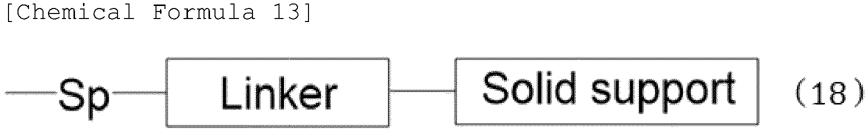

In formula (18), Sp represents a spacer.

The Spacer (Sp) is exemplified by a group having a structure represented by the following formula (19).

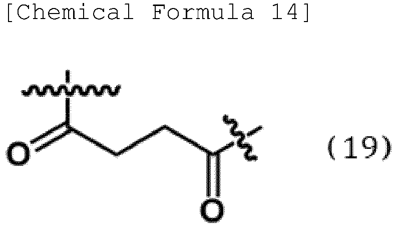

The linker may be, for example, a structure shown in the following formula (20), or a structure in which a hexamethyleneamino group portion in formula (20) is absent and an aminopropyl group is bonded to Si. Alternatively, the linker may be a structure shown in the following formula (21). 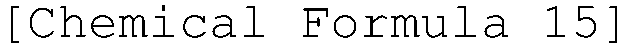 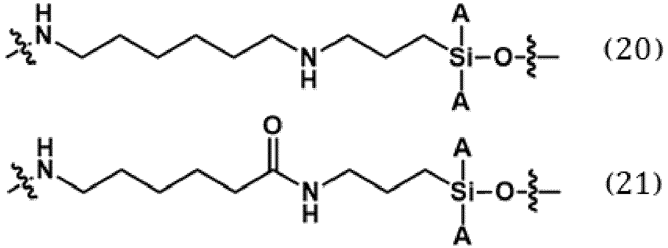 (wherein,
A may be a hydroxy group, an alkoxy group, or an alkyl group. Examples of the alkoxy group include a methoxy group and an ethoxy group. Examples of the alkyl group include a methyl group, an ethyl group, an isopropyl group, and an n-propyl group. Si indicates that it is bonded to the oxygen of a hydroxy group on the surface of a support.)
Examples of the solid support include an inorganic porous support and an organic resin support, and the others. Examples of the inorganic porous support include Controlled Pore Glass (CPG) and zeolite. Examples of the organic resin support include a support composed of polystyrene.

The method for synthesizing oligonucleotides typically includes the following steps.
(1) a step of deprotecting a hydroxy group at a 5' position of a nucleoside whose hydroxy group is protected and which is bonded to a solid support via a linker;
(2) a step of coupling reaction of the hydroxy group at the 5' position produced in the previous step with an amidite compound to obtain a phosphite triester compound;
(3) a step of oxidizing the phosphite triester produced in the previous step to convert it into a phosphate triester to produce an elongated nucleic acid molecule, or an optional step of converting it into a thiophosphate triester;
(4) a step of synthesizing a nucleic acid molecule on the solid support by repeating, any number of times, a series of reaction cycles composed of the steps (1) to (3), that is, the deprotecting step of the hydroxy group at the 5' position of the produced nucleic acid molecule, the coupling step of the hydroxy group at the 5' position with the amidite compound, and the oxidizing step of the produced phosphite triester;
(5) a step of subjecting the nucleic acid molecule on the solid support produced in the step (4) to a step of cleavage and deprotection to release it from the solid support to produce an oligonucleotide with the protecting group removed therefrom; and
(6) a step of deprotecting a protecting group of 2' hydroxy group of a ribose constituting a nucleic acid molecule.

However, in the method for synthesizing oligonucleotides, a step of capping a hydroxy group at a 5' position where the coupling reaction with the amidite compound did not proceed may be included following the step (2) or (3), and a capping step may be added at any point during the series of reaction cycles constituting the step (4).

The step (5) is more specifically conducted in the order of the following reactions (5-1) and (5-2) for the nucleic acid molecule on the solid support produced in the step (4). Here, the reaction of step (5-1) may optionally be conducted, and the reaction of step (5-2) may be conducted using the method described in JP 4705716 B2. As a result, it is possible to produce an oligonucleotide with the protecting groups removed from the nucleic acid molecule released from the solid support, or an oligonucleotide with the 5' terminal hydroxy group protected.
(5-1) a reaction of deprotecting a protecting group of a hydroxy group at a 5' terminal of a nucleic acid molecule; and
(5-2) a reaction of cleaving and releasing a nucleic acid oligomer from a solid support.

The scheme of the steps (1) to (6) is shown as Scheme A in Figure 1. Among the substituents in the chemical formulas in Scheme A, the definitions of G¹, G², B^{a}, and R are as defined above. Additionally, the definitions of G³, G⁴, G⁵, B^{c}, and R' are as described later. Additionally, in the chemical formulas in Scheme A,
Y is identical to or different from each other and each independently represents an oxygen atom or a sulfur atom,
X represents an R group or an OZ group, where Z is as defined above,
W represents an OZ group when X represents an R group, where Z is as defined above, or W represents an OV group when X represents an OZ group, where V represents a protecting group of a hydroxy group,
W₁ is the W group or a group derived from the W group (e.g., a residue cleaved from the solid support, a deprotected group, etc.),
W₁₀ is the W₁ group or a group derived from the W₁ group (e.g., a residue cleaved from the solid support, a deprotected group, etc.),
X₁ is the X group or a group derived from the X group (e.g., a residue cleaved from the solid support, a deprotected group, etc.),
X₁₀ is the X₁ group or a group derived from the X₁ group (e.g., a residue cleaved from the solid support, a deprotected group, etc.),
n represents an integer from 1 to 300, and
m represents an integer from 1 to 300.

A nucleic acid compound of formula (A5) can be further elongated to any desired chain length using a nucleotide-type or non-nucleotide-type linker by the amidite method and can be used for the production of a nucleic acid compound represented by formula (A5'). After cleaving only a nucleic acid compound from the nucleic acid compound of formula (A5') bonded to a solid support to obtain an oligonucleotide represented by formula (A6), further deprotection can yield an oligonucleotide represented by formula (A7).

The following explains the substituents in each formula.

As for G¹, it can be used without any particular limitation as long as it can function as a protecting group, and publicly known protecting groups used for amidite compounds can be widely used. G¹ is preferably, the protecting group represented by the following formula (14). 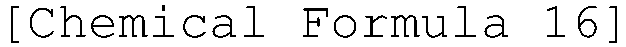 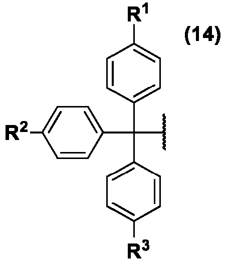 (wherein, R¹, R² and R³ are identical to or different from each other, and each independently represents a hydrogen atom or an alkoxy group.)

One of R¹, R² and R³ is a hydrogen atom, and the remaining two thereof are identical to or different from each other (preferably identical) and represents preferably an alkoxy group, and as the alkoxy group, a methoxy group is particularly preferred. Specifically, G¹ is preferably a 4,4'-dimethoxytrityl group (DMTr group), a 4-monomethoxytrityl group, or a 4,4',4''-trimethoxytrityl group, with the 4,4'-dimethoxytrityl group being particularly preferred.

As for G², it can be used without any particular limitation as long as it can function as a protecting group, and publicly known protecting groups used for amidite compounds can be widely used. Examples of G² include an alkyl group, an alkenyl group, an alkynyl group, a cycloalkyl group, a haloalkyl group, an aryl group, a heteroaryl group, an arylalkyl group, a cycloalkenyl group, a cycloalkylalkyl group, a cyclylalkyl group, a hydroxyalkyl group, an aminoalkyl group, an alkoxyalkyl group, a heterocyclylalkenyl group, a heterocyclylalkyl group, a heteroarylalkyl group, a silyl group, a silyloxyalkyl group, a mono, di or tri- alkylsilyl group, a mono, di or tri- alkylsilyloxyalkyl group, and the others, and these groups may be optionally substituted with one or more electron-withdrawing groups.

G² is preferably an alkyl group substituted with an electron-withdrawing group. Examples of the electron-withdrawing group include a cyano group, a nitro group, an alkylsulfonyl group, a halogen atom, an arylsulfonyl group, a trihalomethyl group, a trialkylamino group, and the others, and preferably a cyano group. As for G², a 2-cyanoethyl group (represented by the following formula) is particularly preferred.

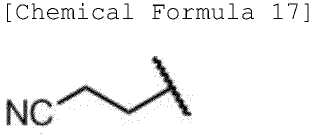

G³ is an alkyl group, and two G³ may be combined with each other to form a cyclic structure. Preferably, both G³ are an isopropyl group.

The alkyl group as the definitions of the above R¹, R², R³, G², and G³ may be a straight chain or a branched chain, and preferably an alkyl group containing 1 to 12 carbon atoms, and more preferably an alkyl group containing 1 to 6 carbon atoms. Specific examples of the alkyl group include methyl, ethyl, n-provyl, isopropyl, n-butyl, isobutyl, tert-butyl, n-pentyl, isopentyl, and hexyl. An alkyl group part which constitutes the alkoxy group in the definition for above substituents has the same definition as that described in the definition of the alkyl group described here.

G⁴ represents a hydrogen atom, an alkali metal ion, an ammonium ion, an alkylammonium ion, or a hydroxyalkylammonium ion. Examples of the alkali metal ion include sodium ion and lithium ion. As the alkyl ammonium ion, specific examples of alkyl part include methyl, ethyl, n-provyl, isopropyl, n-butyl, isobutyl, tert-butyl, n-pentyl, isopentyl, and hexyl, and specific examples thereof include diethyl ammonium ion, triethyl ammonium ion, tetrabutyl ammonium ion, hexyl ammonium ion, and dibutyl ammonium ion, and the others. In addition, as the hydroxyalkylammonium ion, specific examples of hydroxyalkyl part include hydroxymethyl, hydroxyethyl, hydroxy-n-provyl, hydroxy isopropyl, hydroxy-n-butyl, trishydroxymethyl and the others, and more specific examples of the hydroxyalkylammonium ion include trishydroxymethylammonium ion and the others.

G⁵ represents a hydrogen atom or a protecting group, and when it represents a protecting group, it represents the same protecting group as G¹. When G⁵ is deprotected, it is a hydrogen atom, and a nucleotide compound in this case is also subjected to a series of nucleic acid elongation reactions.

In addition, in the method of the present invention, an amidite compound can be used in a free state or a salt state. Examples of the salt of the amidite compound include a base addition salt and an acid addition salt, but which are not particularly limited thereto. Specific examples of the base addition salt include salts with inorganic bases such as sodium salts, magnesium salts, potassium salts, calcium salts, aluminum salts and the others; salts with organic bases such as methylamine, ethylamine, ethanolamine and the others; salts with basic amino acids such as lysine, ornithine, arginine and the others; and ammonium salts. Specific examples of acid addition salts include mineral acids such as hydrochloric acid, hydrobromic acid, hydroiodic acid, sulfuric acid, nitric acid, phosphoric acid and the others; salts with organic acids such as formic acid, acetic acid, propionic acid, oxalic acid, malonic acid, malic acid, tartaric acid, fumaric acid, succinic acid, lactic acid, maleic acid, citric acid, methanesulfonic acid, trifluoromethansulfonic acid, ethanesulfonic acid and the others; and salts with acidic amino acids such as aspartic acid, glutamic acid and the others. Examples of the amidite compounds encompass salts, hydrates, solvates, crystal polymorphs, and the others.

In this description, a nucleobase refers to a group having a natural type or non-natural type nucleobase backbone. The nucleobase also includes modified forms in which the natural type or non-natural type nucleobase backbone is modified.

A nucleobase represented by B^{a} which may be protected by a protecting group is not particularly limited. Examples of the nucleobase include adenine, cytosine, guanine, uracil, thymine, 5-methylcytosine, pseudouracil, 1-methylpseudouracil, and the others. In addition, the nucleobase may be substituted with substituent(s). Examples of the substituent include a halogen atom such as a fluoro group, a chloro group, a bromo group, and an iodo group, an acyl group such as an acetyl group, an alkyl group such as a methyl group and an ethyl group, an arylalkyl group such as a benzyl group, an alkoxy group such as a methoxy group, an alkoxyalkyl group such as a methoxyethyl group, a cyanoalkyl group such as a cyanoethyl group, a hydroxy group, a hydroxyalkyl group, an acyloxymethyl group, an amino group, a monoalkylamino group, a dialkylamino group, a carboxy group, a cyano group, a nitro group and the others, as well as combinations of two or more of these substituents.

As the nucleobase represented by B^{a}, more specifically, the following structures are exemplified. 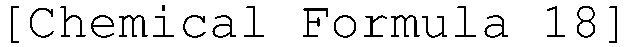 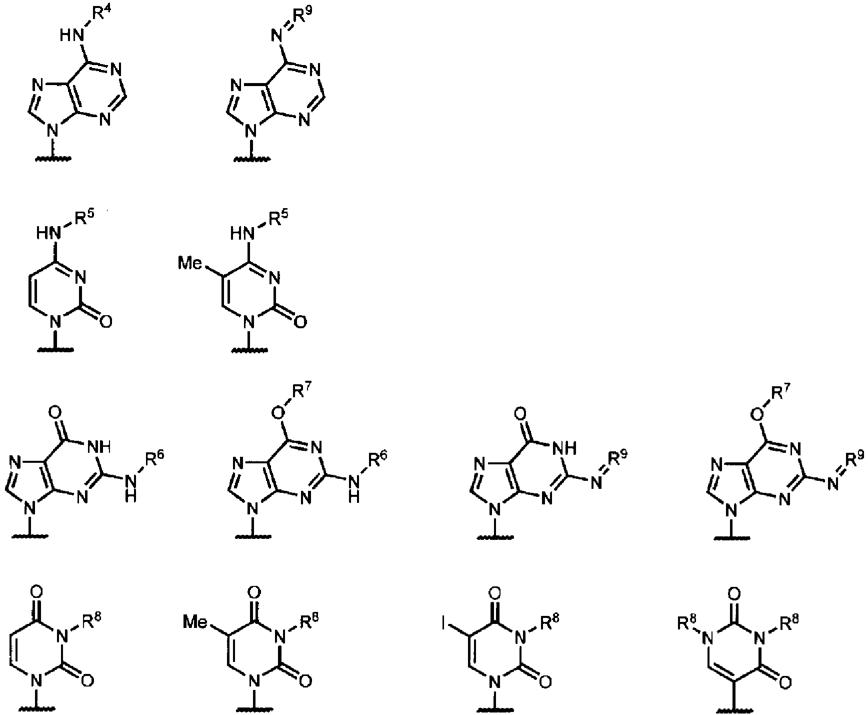 (wherein,
R⁴ represents a hydrogen atom, a methyl group, a phenoxyacetyl group, a 4-tert-butylphenoxyacetyl group, a 4-isopropylphenoxyacetyl group, a phenylacetyl group, an acetyl group, or a benzoyl group,
R⁵ represents a hydrogen atom, an acetyl group, an isobutyryl group, or a benzoyl group,
R⁶ represents a hydrogen atom, a phenoxyacetyl group, a 4-tert-butylphenoxyacetyl group, a 4-isoproylphenoxyacetyl group, a phenylacetyl group, an acetyl group or an isobutyryl group,
R⁷ represents a 2-cyanoethyl group,
R⁸ represents a hydrogen atom, a methyl group, a benzoyl group, a 4-methoxybenzoyl group, or a 4-methylbenzoyl group, and
R⁹ represents a dimethylaminomethylene group.)

When a nucleobase has an amino group outside the ring, a protecting group of the amino group is not particularly limited, and publicly known protecting groups used in nucleic acid chemistry can be used, and examples of the protecting group include a benzoyl group, a 4-methoxybenzoyl group, an acetyl group, a propionyl group, a butyryl group, an isobutyryl group, a phenylacetyl group, a phenoxyacetyl group, a 4-tert-butylphenoxyacetyl group, a 4-isopropylphenoxyacetyl group, and (dimethylamino)methylene group, as well as combinations of two or more of these protecting groups.

B^{c} represents an unprotected nucleobase, but its type is not particularly limited. Examples of the nucleobase include adenine, cytosine, guanine, uracil, thymine, 5-methylcytosine, pseudouracil, 1-methylpseudouracil and the others. In addition, the nucleobase may be substituted with substituent(s). Examples of the substituent include a halogen atom such as a fluoro group, a chloro group, a bromo group, and an iodo group, an acyl group such as an acetyl group, an alkyl group such as a methyl group and an ethyl group, an arylalkyl group such as a benzyl group, an alkoxy group such as a methoxy group, an alkoxyalkyl group such as a methoxyethyl group, a cyanoalkyl group such as a cyanoethyl group, a hydroxy group, a hydroxyalkyl group, an acyloxymethyl group, an amino group, a monoalkylamino group, a dialkylamino group, a carboxy group, a cyano group, a nitro group and the others, as well as combinations of two or more of these substituents.

When R represents a protected hydroxy group, the protecting group can be any that can be used in the amidite method, examples include a 2'-tert-butyldimethylsilyl (TBDMS) group, a 2'-bis(2-acetoxy)methyl (ACE) group, a 2'-(triisopropylsilyloxy)methyl (TOM) group, a 2'-(2-cyanoethoxy)ethyl (CEE) group, a 2'-(2-cyanoethoxy)methyl (CEM) group (WO 2006/022323 A1), a 2'-para-toluylsulfonylethoxymethyl (TEM) group, a 2'-EMM group (WO 2013/027843 A1), and those described in WO 2019/208571 A1. Among these 2' protecting groups for ribonucleoside (RNA), a protecting group represented by formula (10) is exemplified as a preferred protecting group. More preferably, a protecting group represented by formula (15), having a cyano group as an electron-withdrawing group represented by E_{W}, is exemplified. 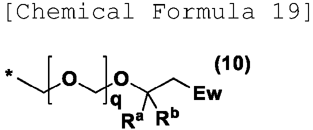 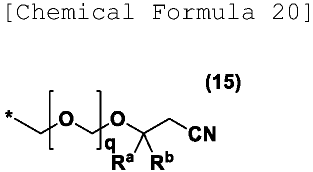 (wherein,
q represents an integer from 0 to 5,
R^{a} and R^{b} are identical to or different from each other and each represents a methyl group, an ethyl group, or a hydrogen atom,
the bond marked with * is bonded to the oxygen of the 2' hydroxy group, and
E_{W} represents an electron-withdrawing group.)

The protecting group represented by formula (15) can be synthesized according to the descriptions in WO 2013/027843 A1 and WO 2019/208571 A1, for example. Amidite compounds having the protecting group can be used for the production of oligonucleotides.

Nucleotides and amidites wherein an R group is a substituent other than a hydroxy group can be produced from nucleosides synthesized by known methods described in JP 3745226 B2 and so on, or WO 2001/053528 A1, JP 2014-221817 A1, or known methods cited therein. Additionally, they can be produced using commercially available products according to the methods described in the examples below or by appropriately modifying these methods.

R' represents a hydroxy group, a hydrogen atom, a fluorine atom, a methoxy group, a 2-methoxyethyl group, or an OQ' group, and Q' represents a methylene group which is bonded to the carbon atom at the 4' position of the ribose, an ethylene group which is bonded to the carbon atom at the 4' position of the ribose, or an ethylidene group which is bonded to the carbon atom at the 4' position of the ribose.

The synthesis of oligonucleotides, except for the coupling step using 2'-OMe-U amidite related to the present invention, can generally be conducted by repeating the deprotecting step and the coupling step according to known methods (for example, the methods described in JP 5157168 B2 or JP 5554881 B2), thereby performing the nucleic acid elongation reaction. The following explains each step.

### (Nucleic Acid Elongation Reaction)

In this description, the "nucleic acid elongation reaction" refers to a reaction that elongates a nucleic acid molecule by sequentially connecting nucleotides via phosphodiester bonds. The nucleic acid elongation reaction can be conducted according to the general amidite method (phosphoramidite method). The nucleic acid elongation reaction may be conducted using a nucleic acid automatic synthesizer that employs the amidite method or may be conducted in liquid-phase synthesis.

The chain length of the oligonucleotides can be, for example, 20 mer or more, 40 mer or more, 50 mer or more, 60 mer or more, 80 mer or more, 100 mer or more, 200 mer or more, 2 to 300 mer, 2 to 250 mer, 2 to 200 mer, 10 to 300 mer, 10 to 250 mer, 10 to 200 mer, 10 to 150 mer, 15 to 300 mer, 15 to 250 mer, 15 to 200 mer, 15 to 150 mer, or 15 to 110mer.

The deprotecting step in the step (1) is a step of deprotecting a protecting group of a 5' hydroxy group at RNA chain terminal which is supported on a solid support (see Scheme A in Figure 1). As a general protecting group, a 4,4'-dimethoxytrityl group (DMTr group), a 4-monomethoxytrityl group, and a 4,4',4"-trimethoxytrityl group are used. Deprotection can be conducted using an acid. Examples of the acid for deprotection include trifluoroacetic acid, dichloroacetic acid, trifluoromethanesulfonic acid, trichloroacetic acid, methanesulfonic acid, hydrochloric acid, acetic acid, p-toluenesulfonic acid, and the others.

The coupling step in the step (2) is a reaction where a nucleoside phosphoramidite represented by the following formula (A3) described in Scheme A in Figure 1 is attached to the 5' hydroxy group at the oligonucleotide chain terminal deprotected in the above deprotecting step. The phosphoramidite represented by following formula (A3) used for the nucleic acid elongation include those where R is a protected hydroxy group, 2'-OMe, 2'-F, 2'-O-tert-butyldimetylsilyl group, 2'-O-methoxyethyl group, 2'-H, 2'-fluoro-2'-deoxy-β-D-arabinofuranosyl and the others. As the above nucleoside phosphoramidite, those where 5' hydroxy group is protected with a protecting group (for example, DMTr group) are used. The coupling step can be conducted by using an activator which activates the above-mentioned nucleoside phosphoramidite. Examples of the activator include 5-benzylthio-1H-tetrazole (BTT), 1H-tetrazole, 4,5-dicyanoimidazole (DCI), 5-ethylthio-1H-tetrazole (ETT), N-methyl benzimidazoliumtriflate (N-MeBIT), benzimidazoliumtriflate (BIT), N-phenylimidazoliumtriflate (N-PhIMT), imidazoliumtriflate (IMT), 5-nitrobenzimidazoliumtriflate (NBT), 1-hydroxybenzotriazole (HOBT), 5-(bis-3,5-trifluoromethylphenyl)-1H-tetrazole, and the others, and 5-benzylthio-1H-tetrazole (BTT) is preferred.

The nucleoside phosphoramidite represented by the formula (A3) described in Scheme A in Figure 1 (hereinafter referred to as amidite) is as follows.
Formula (A3): 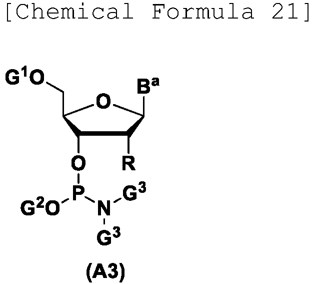 (wherein,
G¹, G², G³, B^{a}, and R are as described above.)

In the present invention, as the amidite of formula (A3), 2'-OMe-U amidite (compound represented by formula (4)) is used in at least one coupling step. Then, in this coupling step, a solution in which 2'-OMe-U amidite is dissolved in a mixed solution containing acetonitrile and an aromatic hydrocarbon represented by formula (3) can be used. An activator may be added to the mixed solution. The activator may be dissolved in the mixed solution.

After the coupling step, as needed, an unreacted 5' hydroxy group may be capped. The capping can be conducted by using publicly known capping solutions such as an acetic anhydride-tetrahydrofuran solution, a phenoxyacetic anhydride/N-methylimidazole solution, and the others.

The oxidizing step in the step (3) is a step of converting a phosphite group which is formed in the above coupling step into a phosphate group or a thiophosphate group. This step is a reaction of converting a trivalent phosphorus into a pentavalent phosphorus using an oxidizing agent, which can be conducted by reacting an oxidizing agent with oligonucleic acid derivatives supported on a solid support.

When a phosphite group is converted into a phosphate group, as "oxidizing agent", for example, iodine can be used. The oxidizing agent can be used by adjusting to 0.005 to 2 M concentration. Water can be used as the oxygen source for oxidation, and pyridine, N-methylimidazole (NMI), N-methylmorpholine, or triethylamine can be used as the base to proceed the reaction. In addition, as for solvent, it is not particularly limited as long as it does not participate in the reaction, but acetonitrile, tetrahydrofuran (THF), or a mixture of these in any ratio can be used. For example, iodine/water/pyridine/acetonitrile, iodine/water/pyridine, iodine/water/pyridine/NMI, or iodine/water/pyridine/THF can be used. The reaction temperature is preferably 5°C to 50°C. The reaction time is usually suitable between 1 minute and 30 minutes. The amount of reagent used is preferably 1 to 100 mol per 1 mol of the compound supported on the solid support, more preferably 1 to 10 mol.

When a phosphite group is converted into a thiophosphate group, as "oxidizing agent", for example, sulfur, 3H-1,2-benzodithiol-3-one-1,1-dioxide (Beaucage reagent), 3-amino-1,2,4-dithiazole-5-thione (ADTT), 5-phenyl-3H-1,2,4-dithiazole-3-one (POS), [(N,N-dimethylaminomethylidene)amino]-3H-1,2,4-dithiazoline-3-thione (DDTT), and phenylacetyldisulfide (PADS) can be also used. The oxidizing agent can be used by diluting it with an appropriate solvent so as to adjust to 0.001 to 2 M concentration. The solvent to be used in the reaction is not particularly limited as long as it does not participate in the reaction, and include, for example, dichloromethane, acetonitrile, pyridine, or any mixed solvents of these solvents. The oxidizing step may be conducted after the above-mentioned capping procedure, or conversely, the capping procedure may be conducted after the oxidizing step, and the order of the procedures are not limited.

In the step (5), a step of deprotecting a phosphate protecting group involves applying an amine compound to deprotect a protecting group in the phosphate portion after the synthesis of the nucleic acid with the desired sequence is completed. Examples of the amine compound include, for example, diethylamine and the others as described in JP 4705716 B2.

The protecting group of the 5' hydroxy group of the nucleoside incorporated in the last stage of the elongation may be used for the column purification with the 5' protecting group as a tag after the below-mentioned procedures of cleaving from a solid support and deprotecting of a protecting group, or alternatively, the protecting group of the 5' hydroxy group may be deprotected after the column purification.

In the step (5), the cleavage of the oligonucleotide, which has been elongated to the desired chain length on the solid support, from the solid support is typically conducted by using concentrated aqueous ammonia as a cleaving agent.

Further, using ammonia or an amine compound or the others, for example, an oligonucleotide chain is collected by cleaving from a solid support. Examples of the amine compound include methylamine, ethylamine, isopropylamine, ethylenediamine, diethylamine, and the others.

In the step (6), the protecting group of the 2'-hydroxy group of the ribose in the nucleic acid compound (A6) cleaved from the solid support in the step (5) can be removed according to the methods described in WO 2006/022323 A1, WO 2013/027843 A1, or WO 2019/208571 A1, to obtain the deprotected oligonucleotide (A7).

Examples of the oligonucleotide which can be produced according to the production method of the present invention include those wherein a nucleoside contained in the oligonucleotide is RNA, DNA, RNA containing 2'-O-MOE, 2'-O-Me, or 2'-F, and LNA, which is not limited thereto. For example, various nucleosides described in Xiulong, Shen et al., Nucleic Acids Research, 2018, Vol. 46, No.46, 1584-1600, and Daniel O'Reilly et al., Nucleic Acids Research, 2019, Vol. 47, No.2, 546-558 are included.

As typical examples of oligonucleotides which can be used in the production method of the present invention, the following examples are indicated in addition to examples described in working examples, which are not limited thereto.

Hereinafter, in a description of a sequence, U represents uridine (ST.25 format), C represents cytidine, A represents adenosine, and G represents guanosine. Additionally, T in the ST.26 format represents uridine.

Oligonucleotides having the following sequences (A) and (B) as described in WO 2019/060442 A1 are exemplified.
Sequence (A): 5' -AUGGAAUmACUCUUGGUUmACdTdT-3' (conforms to ST.25 format) (5'-ATGGAATmACTCTTGGTTmACdTdT-3' (conforms to ST.26 format)) (Antisense) (Sequence No. 1) 21 mer
Sequence (B): 5'-GUmAACmCmAAGAGUmAUmUmCmCmAUmdTdT (conforms to ST.25 format) (5'-GTmAACmCmAAGAGTmATmTmCmCmATmdTdT-3' (conforms to ST.26 format)) (Sense) (Sequence No. 2) 21 mer

In the sequences (A) and (B), Um represents 2'-O-methyluridine (ST.25 format), Tm represents 2'-O-methyluridine (ST.26 format), Cm represents 2'-O-methylcytidine, and dT represents thymidine. As described herein, unless stated otherwise, the abbreviations in the sequence may be applied to both of the ST.25 format and the ST.26 format.

An oligonucleotide as described in JP 2017-537626 A1 is exemplified. A typical example is an oligonucleotide having the following sequence (C).
Sequence (C): 5'-AmsGmsUmsCCUCAUCUCCCUCAAGCGUUUAAGAGCUAUGCUGGUAACAGCAUAGCAAG UUUAAAUAAGGCUAGUCCGUUAUCAACUUGAAAAAGUGGCACCGAGUCGGUGCUUUUms UmsUmsU-3' (conforms to ST.25 format) ((5'-AmsGmsTmsCCTCATCTCCCTCAAGCGTTTAAGAGCTATGCTGGTAACAGCATAGCAAG TTTAAATAAGGCTAGTCCGTTATCAACTTGAAAAAGTGGCACCGAGTCGGTGCTTTTms TmsTmsT-3' (conforms to ST.26 format)) (Sequence No. 3) 113 mer

In the sequence (C), "Um" represents 2'-O-methyluridine (ST.25 format), "Tm" represents 2'-O-methyluridine (ST.26 format), "Am" represents 2'-O-methyladenosine, "Gm" represents 2'-O-methylguanosine, and "s" represents phosphorothioate modification.

### EXAMPLES

Hereinafter, the present invention is explained in more detail by working examples, but the technical scope of the present invention is not limited to these examples.

### Measurement methods

First, various measurement methods used in the following tests are shown below.

### (Measurement method 1: Measurement of n-1 mer impurities and FLP ratio in the oligonucleotide)

The purity of the oligonucleotide was measured using HPLC. FLP stands for the target material (Full Length Product).

The HPLC measurement conditions are shown in Table 1 below.

### [Table 1]

**Table 1**

| | |
|---|---|
| Column | DNAPac^{™} PA200 4 × 250mm |
| Flow rate | 1.0mL/min |
| Detection wavelength | 260nm |
| Mobile phase A | 25mM Tris-HCl buffer (pH=8.0), 10% CH₃CN, 6M Urea water |
| Mobile phase B | 500mM NaClO₄, 25mM Tris-HCl buffer (pH=8.0), 10% CH₃CN, 6M Urea water |
| Gradient condition | Bconc. 10%(0min)-40%(20min)-40%(25min) -90%(25.01min)-90%(29.01min)-10%(45min) |
| Column temperature | 80°C |
| Injection volume | 10µL |

### (Measurement method 2: Measurement of the yield of the oligonucleotide)

OD₂₆₀ of the above crude product was measured. OD₂₆₀ represents an absorbance at UV260 nm per 10 mm optical path length in a 1 mL solution (pH = 7.5). Since it is generally known that 1 OD = 40 µg for RNA, the yield was calculated based on the above measured value of OD₂₆₀.

### Solid-phase synthesis of oligonucleotides

Sequence (I): 5'-UmUmUmUmUmUmUmUmUmUmUmUmUmUmUmUmUmUmUmUmUmUmUmUmUm-3' (conforms to ST.25 format) (5'-TmTmTmTmTmTmTmTmTmTmTmTmTmTmTmTmTmTmTmTmTmTmTmTmTm-3' (conforms to ST.26 format) (Sequence No. 4) 25 mer

In the sequence (I), "Um" represents 2'-O-methyluridine (ST.25 format), and "Tm" represents 2'-O-methyluridine (ST.26 format).

The sequence (I) represents the following structure (16).

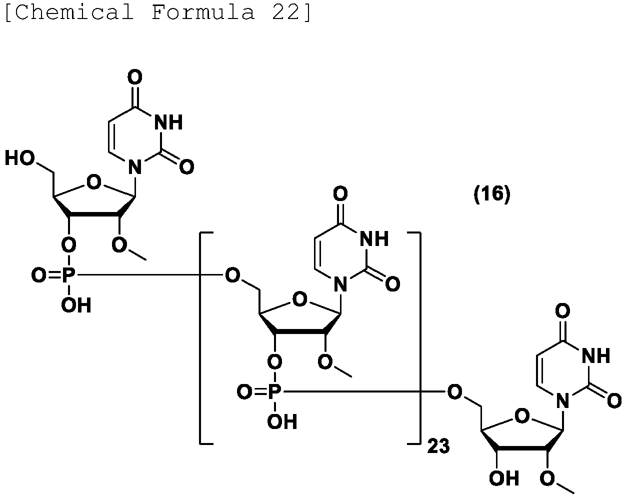

Using Controlled Pore Glass (CPG) as a solid support and an AKTA oligopilot plus 100 (manufactured by GE healthcare) as a nucleic acid synthesizer, according to the phosphoramidite solid-phase synthesis method, an oligonucleotide composed by the above sequence (I) was synthesized from the 3' side to the 5' side. The synthesis was conducted on a scale of about 53 µmol. In addition, for the synthesis, a 2'-OMe-U amidite solution, in which 2'-OMe-U amidite represented by formula (4) was dissolved in any solvent, was used, and a high-purity dichloroacetic acid toluene solution was used as a deblocking solution, and a 5-benzylthio-1H-tetrazole solution was used as a coupling agent, and an iodine solution was used as an oxidizing agent, and a phenoxyacetic anhydride solution and an N-methylimidazole solution were used as capping solutions.

Next, specific production examples of oligonucleotides produced by the production method of the present invention are shown. Here, in the following examples, the oligonucleotides produced by the production method of the present invention are oligonucleotides having sequence (I) shown by sequence No. 4.

In addition, 2'-OMe-U derivative CPG described in the following Examples and Comparative Examples refers to the compound represented by the following formula (17). However, the circle depicted in formula (17) schematically represents CPG.

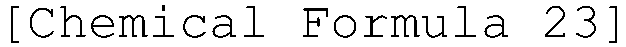

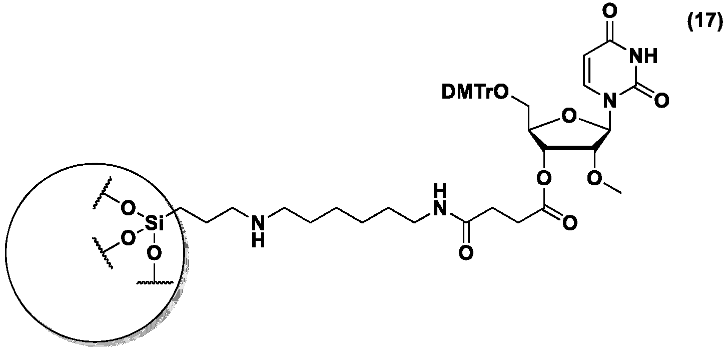

### (Example 1)

Using 53.7 µmol of 2'-OMe-U derivative CPG and a 2'-OMe-U amidite solution adjusted to 75 mM with acetonitrile: toluene 9:1 as the solvent, an oligonucleotide shown in sequence (I) was automatically synthesized from the 3' end to the 5' end using an AKTA oligopilot plus 100 (manufactured by GE Healthcare). The procedure for automatic synthesis is, first, a 3% dichloroacetic acid toluene solution was liquid-transferred to the CPG to deprotect a trityl protecting group at the 5' position, then, a 2'-OMe-U amidite solution and a 5-benzylmercapto-1H-tetrazole acetonitrile solution as a coupling agent were liquid-transferred to the CPG to proceed with the coupling reaction at the 5' hydroxy group. Next, a 50 mM iodine solution was liquid-transferred to convert a phosphite group to a phosphate group. Next, using a 0.1 M phenoxyacetic anhydride acetonitrile solution and a 10 wt% N-methylimidazole/10 wt% 2,6-lutidine acetonitrile solution as capping solutions, capping was conducted at the reaction sites where the coupling reaction did not proceed. These steps were repeated a total of 24 times to synthesize a nucleic acid oligonucleotide with the sequence shown in sequence (I) on the CPG support. After that, a trityl protecting group at the 5' position was deprotected with a 3% dichloroacetic acid toluene solution. After that, for a CPG support carrying 8.0 µmol of an oligonucleotide, using 4.09 g of ammonia water and 1.21 g of ethanol, the oligonucleotide was released from the solid support. After that, the solid support was removed by filtration, and ammonia water and ethanol were removed by vacuum drying, the desired oligonucleotide was obtained as a dry solid. As a result of measurement by Measurement method 2, the yield was 57.3 mg, and as a result of measurement by Measurement method 1, the FLP ratio was 76.04%, and the n-1 mer ratio was 7.17%.

### (Example 2)

In the method of Example 1, except for using 55.6 µmol of 2'-OMe-U derivative CPG, and acetonitrile:toluene 4:1 as the solvent for the 2'-OMe-U amidite solution, an oligonucleotide of sequence (I) was obtained by the same method. The yield was 58.3 mg, the FLP ratio was 78.01%, and the n-1 mer ratio was 5.24%.

### (Example 3)

In the method of Example 1, except for using 53.5 µmol of 2'-OMe-U derivative CPG, and acetonitrile:toluene 4:6 as the solvent for the 2'-OMe-U amidite solution, an oligonucleotide of sequence (I) was obtained by the same method. The yield was 54.3 mg, the FLP ratio was 74.24%, and the n-1 mer ratio was 4.14%.

### (Example 4)

In the method of Example 1, except for using 53.1 µmol of 2'-OMe-U derivative CPG, and acetonitrile:o-xylene 4:1 as the solvent for the 2'-OMe-U amidite solution, an oligonucleotide of sequence (I) was obtained by the same method. The yield was 58.6 mg, the FLP ratio was 77.84%, and the n-1 mer ratio was 5.22%.

### (Example 5)

In the method of Example 1, except for using 53.1 µmol of 2'-OMe-U derivative CPG, and acetonitrile:chlorobenzene 4:1 as the solvent for the 2'-OMe-U amidite solution, an oligonucleotide of sequence (I) was obtained by the same method. The yield was 57.3 mg, the FLP ratio was 77.82%, and the n-1 mer ratio was 5.78%.

### (Example 6)

In the method of Example 1, except for using 52.4 µmol of 2'-OMe-U derivative CPG, and acetonitrile:o-dichlorobenzene 4:1 as the solvent for the 2'-OMe-U amidite solution, an oligonucleotide of sequence (I) was obtained by the same method. The yield was 56.4 mg, the FLP ratio was 76.62%, and the n-1 mer ratio was 5.50%.

### (Comparative Example 1)

In the method of Example 1, except for using 52.8 µmol of 2'-OMe-U derivative CPG, and acetonitrile:dichloromethane 4:1 as the solvent for the 2'-OMe-U amidite solution, an oligonucleotide of sequence (I) was obtained by the same method. The yield was 59.4 mg, the FLP ratio was 76.87%, and the n-1 mer ratio was 7.68%.

The results of the FLP ratio and the n-1 mer ratio for Examples 1 to 6 and Comparative Example 1 are shown in Table 2.

### [Table 2]

**Table 2**

| Experiment name | Solvent type | Solvent mixture ratio (Volume ratio) | n-1 mer ratio | FLP ratio | n-1 mer /FLP | Relative comparison with comparative example |
|---|---|---|---|---|---|---|
| Example 1 | acetonitrile-toluene | 9:1 | 7.17% | 76.04% | 0.094 | 94% |
| Example 2 | acetonitrile-toluene | 4:1 | 5.24% | 78.01% | 0.067 | 67% |
| Example 3 | acetonitrile-toluene | 2:3 | 4.14% | 74.24% | 0.056 | 56% |
| Example 4 | acetonitrile-o-xylene | 4:1 | 5.22% | 77.84% | 0.067 | 67% |
| Example 5 | acetonitrile-chlorobenzene | 4:1 | 5.78% | 77.82% | 0.074 | 74% |
| Example 6 | Acetonitrile-o-dichlorobenzene | 4:1 | 5.50% | 76.62% | 0.072 | 72% |
| Comparative Example 1 | acetonitrile-dichloromethane | 4:1 | 7.68 | 76.87% | 0.100 | 100% |

From the results in the above Table 2, it was found that when acetonitrile and aromatic hydrocarbons are used as the solvent for the 2'-OMe-U amidite solution, the n-1 mer impurities can be relatively reduced compared to the case where the solution of Comparative Example 1 is used.

### [Free text of Sequence Listing]

Sequence Nos. 1 to 4 in Sequence Listing represent base sequences of oligonucleotides that are produced according to the production method of the present invention.

[Sequence Listing]

## Claims

1. A production method of an oligonucleotide comprising a step of coupling a compound represented by formula (2): 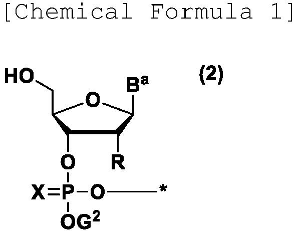 (wherein,
G² represents a protecting group of a hydroxy group,
B^{a} represents a nucleobase which may be protected by a protecting group,
R represents a protected hydroxy group, a hydrogen atom, a fluorine atom, a methoxy group, a 2-methoxyethyl group, or an OQ' group,
Q' represents an alkylene group which is bonded to the carbon atom at the 4' position of the ribose,
X represents an oxygen atom or a sulfur atom, and
the bond marked with * represents a bond to the 3' terminal side of a nucleic acid.)
with 2'-OMe-U amidite represented by formula (4): 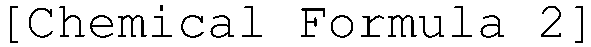 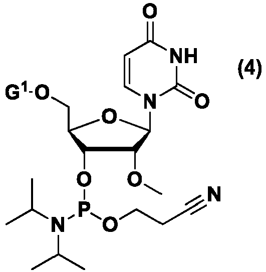 (wherein,
G¹ represents a protecting group of a hydroxy group.) in the presence of an activator, wherein the 2'-OMe-U amidite is dissolved in a mixed solution containing acetonitrile and an aromatic hydrocarbon represented by formula (3): 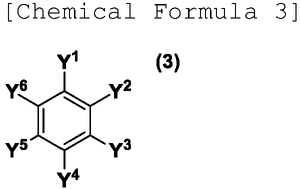 (wherein, Y¹ to Y⁶ are identical to or different from each other and each independently represents a hydrogen atom, a methyl group, an ethyl group, or a halogen atom.).

2. The production method of the oligonucleotide according to claim 1 wherein the aromatic hydrocarbon represented by formula (3) is benzene having one or two substituents selected from the group consisting of a methyl group, an ethyl group, and a halogen atom, and when benzene has two substituents, the substituents may be the same or different.

3. The production method of the oligonucleotide according to claim 1 wherein the aromatic hydrocarbon represented by formula (3) is selected from the group consisting of toluene, o-xylene, m-xylene, p-xylene, chlorobenzene, o-dichlorobenzene, m-dichlorobenzene, p-dichlorobenzene, and mixtures of two or more of these.

4. The production method of the oligonucleotide according to any one of claims 1 to 3 wherein the volume ratio of acetonitrile to the aromatic hydrocarbon represented by formula (3) in the mixed solution in which 2'-OMe-U amidite represented by formula (4) is dissolved is 99:1 to 1:99 in acetonitrile: the aromatic hydrocarbon represented by formula (3).

5. The production method of the oligonucleotide according to any one of claims 1 to 3 wherein the volume ratio of acetonitrile to the aromatic hydrocarbon represented by formula (3) in the mixed solution in which 2'-OMe-U amidite represented by formula (4) is dissolved is 90:10 to 10:90 in acetonitrile: the aromatic hydrocarbon represented by formula (3).

6. The production method of the oligonucleotide according to any one of claims 1 to 3 wherein the volume ratio of acetonitrile to the aromatic hydrocarbon represented by formula (3) in the mixed solution in which 2'-OMe-U amidite represented by formula (4) is dissolved is 90:10 to 40:60 in acetonitrile: the aromatic hydrocarbon represented by formula (3).

7. The production method of the oligonucleotide according to any one of claims 1 to 6 wherein the concentration of 2'-OMe-U amidite in the 2'-OMe-U amidite solution represented by formula (4) is 0.01 to 0.4 M.

8. The production method of the oligonucleotide according to any one of claims 1 to 6 wherein the concentration of 2'-OMe-U amidite in the 2'-OMe-U amidite solution represented by formula (4) is 0.05 to 0.2 M.

9. The production method of the oligonucleotide according to any one of claims 1 to 8 wherein the activator is 5-benzylthio-1H-tetrazole.

10. The production method of the oligonucleotide according to any one of claims 1 to 9 wherein when R in the compound represented by formula (2) is a protected hydroxy group, the protecting group of the hydroxy group is a protecting group represented by formula (10): 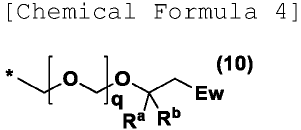 (wherein,
q represents an integer from 0 to 5,
R^{a} and R^{d} are identical to or different from each other and each represents a methyl group, an ethyl group, or a hydrogen atom,
the bond marked with * is bonded to the oxygen of the 2' hydroxy group, and
Ew represents an electron-withdrawing group.).

11. The production method of the oligonucleotide according to any one of claims 1 to 10 wherein in 2'-OMe-U amidite represented by formula (4), G¹ is a 4,4'-dimethoxytrityl group.

12. The production method of the oligonucleotide according to any one of claims 1 to 11 conducted by solid-phase synthesis method.

13. A solution containing 2'-OMe-U amidite represented by formula (4): 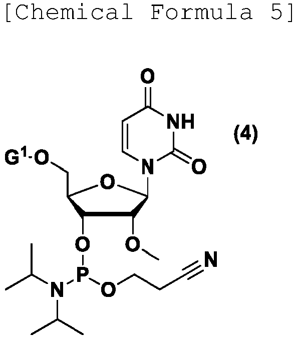 (wherein,
G¹ represents a protecting group of a hydroxy group.) acetonitrile, and an aromatic hydrocarbon represented by formula (3): 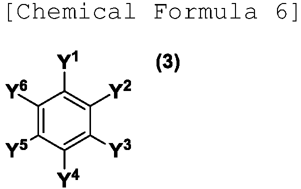 (wherein, Y¹ to Y⁶ are identical to or different from each other and each independently represents a hydrogen atom, a methyl group, an ethyl group, or a halogen atom.).

14. A method of using the solution described in claim 13 containing 2'-OMe-U amidite represented by formula (4), acetonitrile, and the aromatic hydrocarbon represented by formula (3) for a production of an oligonucleotide.
